# EUROPÄISCHE PATENTANMELDUNG

(11) **EP 1 674 053 A1**
(43) Veröffentlichungstag der Anmeldung: **28.06.2006**
(21) Anmeldenummer: 05109193.2
(22) Anmeldetag: 04.10.2005
(51) Int. Cl.: A61F 2/44

(54) **Implantat zum Ersetzen von Knochen oder Knorpel, mit Fixierglied**

(30) Priorität: 22.12.2004 CH 21232004
(71) Anmelder: Hermann, Werner, CH-6312 Steinhausen (CH)
(72) Erfinder: Matzen, Klaus, 86356, Neusäss (DE)
(74) Vertreter: OK pat AG

(57) **Zusammenfassung**

Implantat zum Ersetzen mindestens eines Teils eines Knochens oder Knorpels eines Patienten. Das Implantat (10) umfasst einen Implantat-Körper (40) und eine Fixiereinrichtung mit mindestens einem Fixierglied (50). Das Fixierglied (50) ist aus einer Ruhelage in eine Wirklage bewegbar, wobei es sich schneiden in einem dem Implantatkörper benachbarten Knochen bewegt. In der Wirklage greift das Fixierglied (50) in den Knochen ein. Die Fixiereinrichtung weist eine Stellvorrichtung (60) auf. Die Stellvorrichtung (60) ist bewegungsmässig mit dem Fixierglied (50) gekoppelt, und sie ist verstellbar zwischen einer Passivstellung, in der das Fixierglied (50) seine Ruhelage einnimmt, und einer Aktivstellung, in der das Fixierglied (50) eine Wirklage einnimmt.

## Beschreibung

Die Erfindung betrifft ein Implantat nach dem Oberbegriff des Anspruchs 1.

Implantate treten an die Stelle beschädigter oder fehlender Knochenteile, Knochen, Knorpelteile oder Knorpel.

Implantate der eingangs genannten Art werden beispielsweise als Bandscheiben-Implantate bzw. Bandscheiben-Ersatz verwendet und als solche mit 'Cage' bezeichnet, es sind aber auch andere Applikationen möglich, bei welchen ein Implantat fest mit einem flächigen Knochenbereich verbunden wird. Implantate werden hierbei als Ersatz für eine Bandscheibe oder für einen Teil einer Bandscheibe zwischen zwei Wirbelkörpern eingefügt und an einem oder beiden der angrenzenden Wirbelkörper fixiert.

Herkömmliche als Bandscheiben-Ersatz einsetzbare Implantate sind unterschiedlich ausgebildet. Bekannt sind beispielsweise Implantate, die aus einem Implantat-Körper und einer Fixiereinrichtung bestehen. Die Fixiereinrichtung lässt sich aus einer Ruhelage, die sie vor und zu Beginn der Applikation am Patienten einnimmt, in eine Wirklage bringen, in der sie den Implantatkörper in seiner definitiven Lage fixiert. Als Fixiereinrichtung bzw. Fixierglied wird hierbei eine Schraube benutzt, die im Laufe der Applikation des Bandscheiben-Ersatzes in einen der benachbarten Wirbelkörper eingeschraubt wird.

Die Verwendung einer Schraube als Fixierglied hat mehrere Nachteile. Es ist ungünstig, dass durch das Einschrauben in den Wirbelkörper verhältnismässig viel Knochenmaterial entfernt werden muss, was an und für sich unerwünscht ist und die Gefahr mit sich bringt, dass Knochensplitter im Körper verbleiben. Muss das Implantat zu einem späteren Zeitpunkt wieder entfernt werden, beispielsweise weil es nicht die erhoffte Wirkung hat oder Beschwerden hervorruft, so ist das Lockern der Schraube sehr schwierig. Ausserdem hinterlässt die entfernte Schraube ein mindestens zeitweilig bestehendes Schraubenloch im Knochen zurück, in dessen Umgebung kein weiteres Fixierglied eines neuen Implantates angebracht werden kann.

Die Aufgabe der Erfindung wird demzufolge darin gesehen, ein Implantat der eingangs genannten Art vorzuschlagen, mit welchem die Nachteile des Standes der Technik vermieden werden können.

Die Lösung dieser Aufgabe erfolgt erfindungsgemäss durch die Merkmale des Patentanspruchs 1. Bevorzugte Weiterbildungen des neuen Implantates sind durch die abhängigen Patentansprüche umschrieben.

Das neue Implantat weist somit einen Implantatkörper und eine Fixiereinrichtung auf. Die Fixiereinrichtung umfasst mindestens ein Fixierglied und eine Stellvorrichtung für das Fixierglied. Die Stellvorrichtung ist bewegungsmässig mit dem Fixierglied gekoppelt, und sie ist zwischen einer Passivstellung und Aktivstellungen verstellbar. Ist die Stellvorrichtung in ihrer Passivstellung, so nimmt das Fixierglied seine Ruhelage ein. Wird die Stellvorrichtung in eine Aktivstellung verstellt, so gelangt das Fixierglied in eine Wirklage, in der es in einen benachbarten Knochen eingreift.

Vorzugsweise ist die gesamte Fixiereinrichtung, mindestens aber das Fixierglied, integral mit dem Implantat-Körper gebildet.

Es hat sich als günstig erwiesen, das Fixierglied als Lamelle mit einer Lamellenfläche auszubilden. Die Lamelle weist mindestens eine Schneidkante auf. Die Schneidkante läuft beim Bewegen des Fixiergliedes vor und schneidet sich hierbei in den Knochen ein, bis das Fixierglied bzw. die Lamelle die endgültige Lage im Knochen erreicht hat.

Besonders vorteilhaft ist es, das als Lamelle ausgebildete Fixierglied durch Drehung um eine Achse, die im wesentlichen senkrecht zur Lamellenfläche gerichtet ist, zu bewegen. Bei einer solchen Anordnung besitzt die Lamelle vorzugsweise eine erste Schneidkante, die vorläuft, wenn das Fixierglied durch Drehung in einer Richtung seine Wirklage gebracht und, und eine zweite Schneidkante, die vorläuft, wenn das Fixierglied durch Drehung in einer Gegenrichtung in seine Ruhelage zurückgebracht wird, entweder weil es zu weit gedreht wurde oder weil das ganze Implantat entfernt werden soll.

Die Fixiereinrichtung kann mehrere mit demselben Knochen in Eingriff bringbare Fixierglieder umfassen. Hierbei können einzelne Fixierglieder durch separate oder gemeinsame Stellvorrichtungen verstellbar sein.

Die Stellvorrichtung weisen vorzugsweise einen zulaufenden Abschnitt auf, um beim Verstellen des Stellgliedes das Fixierglied zu verschieben. Insbesondere kann das Stellglied eine Stellschraube sein, die einen zulaufenden Abschnitt besitzt.

Es hat sich als günstig erwiesen, Sicherungselemente vorzusehen, um mittelbar bzw. unmittelbar die Stellvorrichtung in ihrer definitiven Aktivlage bzw. die Fixierglieder in ihrer Wirklage zu sichern.

Das Implantat kann weitere gleich oder ähnliche Fixiereinrichtungen aufweisen, deren Fixierglied dazu bestimmt sind, in einen weiteren angrenzenden Knochen oder Knochenteil einzugreifen. Damit kann das Implantat zwischen zwei aufeinanderfolgenden Knochen fixiert werden, beispielsweise als Bandscheiben-Ersatz zwischen zwei aufeinanderfolgenden Wirbelkörpern.

Wenn der Implantat-Körper und ggfs. das mindestens eine Fixierglied eine strukturierte Aussenfläche aufweisen, so wird dadurch das Einwachsen im Körper eines Patienten bzw. das Umwachsen der Implantat-Bestandteile mit körpereigner Substanz gefördert. Implantat-Körper, deren Fixierglieder strukturierte Aussenflächen haben, sind allerdings schwieriger zu montieren und zu demontieren. Die Strukturierung der Aussenfläche des Implantat-Körpers selbst sollte so beschaffen sein, dass eine Entfernung des Implantates nicht zu viele Probleme bietet. Als Strukturierung für den Implantat-Körper kann beispielsweise eine oder mehrere den angrenzenden Knochen oder Knochenteilen zugewandte Oberfläche Rillen aufweisen. Werden Fixierglieder aus Lamellen eingesetzt, so verlaufen diese Rillen im montierten Zustand vorzugsweise quer zur Richtung der Lamellenflächen.

Um den Implantat-Körper optimal zu gestalten, kann er unterschiedliche Höhen aufweisen, welche gemäss dem jeweiligen örtlichen Abstand der im montierten Zustand benachbarten Knochen gewählt werden.

Üblicherweise weist der Implantat-Körper Ausbrüche oder Durchbrüche auf. Da Implantat-Körper häufig aus einem Material mit höherem spezifischen Gewicht bestehen als Knochen oder Knorpel, können solche Ausbrüche oder Durchbrüche zur Gewichtsreduktion dienen. Sie erleichtern auch die innige Verbindung des Implantats mit körpereigenen Substanzen, indem neu gebildete Substanz das Implantat umwächst und auch in die Ausbrüche einwächst.

In gewissen Fällen ist es wünschbar, die Verbindung von zwei Knochen oder Knochenteilen durch ein Implantat beweglich zu gestalten. Hierzu kann der Implantat-Körper in ein erstes Implantatkörper-Aussenteil und ein zweites Implantatkörper-Aussenteil aufgeteilt ist, zwischen denen ein Implantatkörper-Mittelteil angeordnet ist, relativ zu welchem die Aussenteile im montierten Zustand bewegbar sind, wobei diese Bewegung üblicherweise begrenzt ist. Hierzu kann von einander gegenüberstehenden Flächen des Aussenteiles und des Mittelteiles mindestens eine Fläche nach aussen konvex und vorzugsweise die andere Fläche nach aussen konkav ausgebildet ist, so dass eine kugelgelenk-artige Anordnung zu Stande kommt. Auch kann das Mittelteil im montierten Zustand elastisch sein.

Die Erfindung wird im Folgenden an Hand von Ausführungsbeispielen und mit Bezug auf die Zeichnung ausführlich beschrieben. Beim diesem Ausführungsbeispiel ist das Implantat ein Bandscheiben-Ersatz, und die nicht-schematisierten Figuren können für diese Anwendung im wesentlichen als massstäblich bzw. mindestens in ausführbaren Proportionen betrachtet werden. Angegebene Zahlenwerte sind aber nur Richtwerte, tatsächliche Grössen können davon in beträchtlichem Masse abweichen, wobei auch die Abmessungen des Patienten eine Rolle spielen können. Es wird aber ausdrücklich darauf hingewiesen, dass die Erfindung keinesfalls auf Bandscheiben-Ersatz oder auf den Ersatz von Knorpelsubstanz beschränkt ist. Es zeigen:
- Fig. 1: ein als Bandscheiben-Ersatz ausgebildetes Implantat nach der Erfindung, zwischen zwei aufeinanderfolgenden Wirbelkörpern, von der Seite gesehen, in stark vereinfachter, schematisierter Darstellung;
- Fig. 2: ein als Bandscheiben-Ersatz ausgebildetes Implantat nach der Erfindung, von der Seite;
- Fig. 3A: den Implantat-Körper des in Fig. 2 dargestellten Implantates, von der Seite;
- Fig. 3B: den in Fig. 2 dargestellten Implantat-Körper, von oben oder unten;
- Fig. 4A: das Fixierglied des in Fig. 2 dargestellten Implantates, von oben;
- Fig. 4B: das in Fig. 4A dargestellte Fixierglied, von der Seite;
- Fig. 4C: das in den Fig. 5A und 5B dargestellte Fixierglied, von vorn;
- Fig. 4D: das in den Fig. 4A bis 4C dargestellte Fixierglied, von hinten;
- Fig. 5A: das Stellglied des in Fig. 2 dargestellten Implantates, von der Seite des Patienten aus gesehen;
- Fig. 5B: das in Fig. 5A dargestellte Stellglied, in einer Seitenansicht des Stellgliedes;
- Fig. 5C: einen Schnitt längs der Linie C-C der Fig. 5B; und
- Fig. 6: einen in mehrere Teile aufgeteilten Implantatkörper.

Es sei vorausgeschickt, dass sich in der nachfolgenden Beschreibung die Begriffe oben, unten, links, rechts, vorne, hinten - wenn nichts anderes erwähnt - auf die Lage eines als Bandscheiben-Ersatz ausgebildeten Implantates nach seiner Einfügung in den Körper eines aufrecht stehenden Patienten bzw. auf den Körper des aufrecht stehenden Patienten beziehen. Gleiche und ähnliche, im wesentlichen gleich wirkende Teile sind in den verschiedenen Figuren mit dem gleiche Bezugszeichen versehen. Gewisse offensichtlich erkennbare Einzelheiten sind nicht beschrieben.

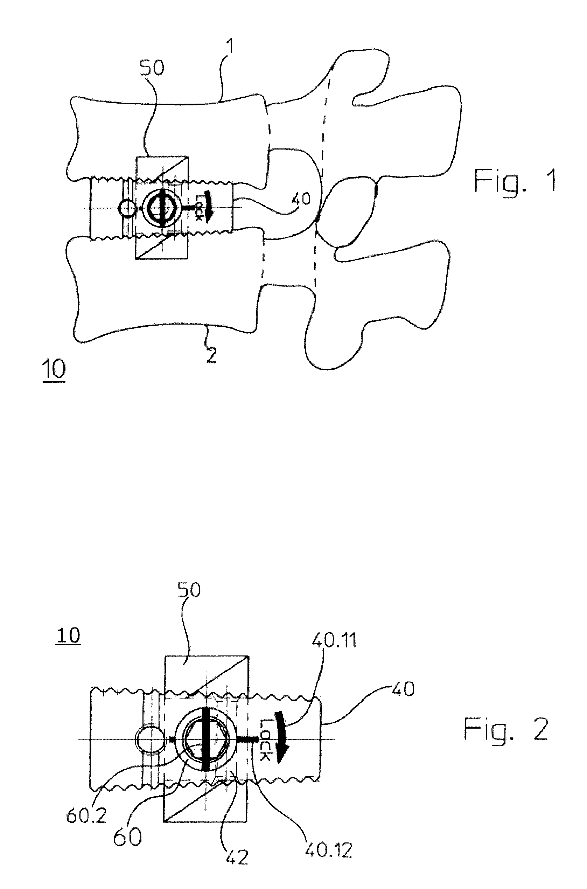

Fig. 1 zeigt ein Implantat 10, das zwischen zwei Knochenteilen 1, 2 angeordnet, aber noch nicht fixiert ist. Die Knochenteile 1 und 2 sind aufeinander folgende Wirbelkörper, und das Implantat 10 ist ein Bandscheiben-Ersatz. Die Wirbelsäule ist bekanntlich je nach Ort nach vorne oder hinten verschieden gebogen, und die Wirbelkörper sind daher nicht vollständig parallel, und im Allgemeinen ist der Abstand zwischen zwei aufeinanderfolgenden Wirbelkörpern nicht konstant. Dieser Tatsache wird hier Rechnung getragen, indem das Implantat 10 nicht eine konstante Höhe aufweist.

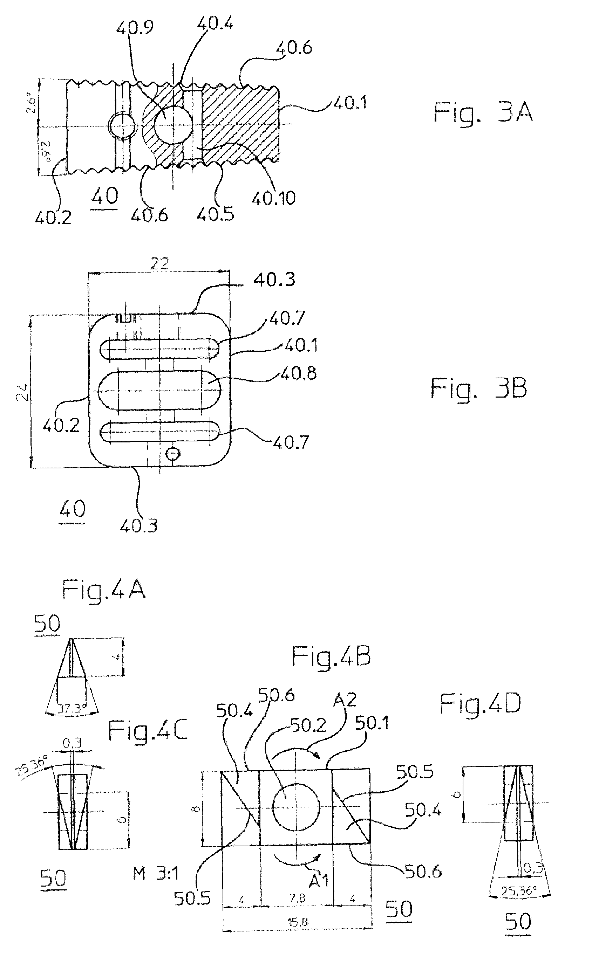

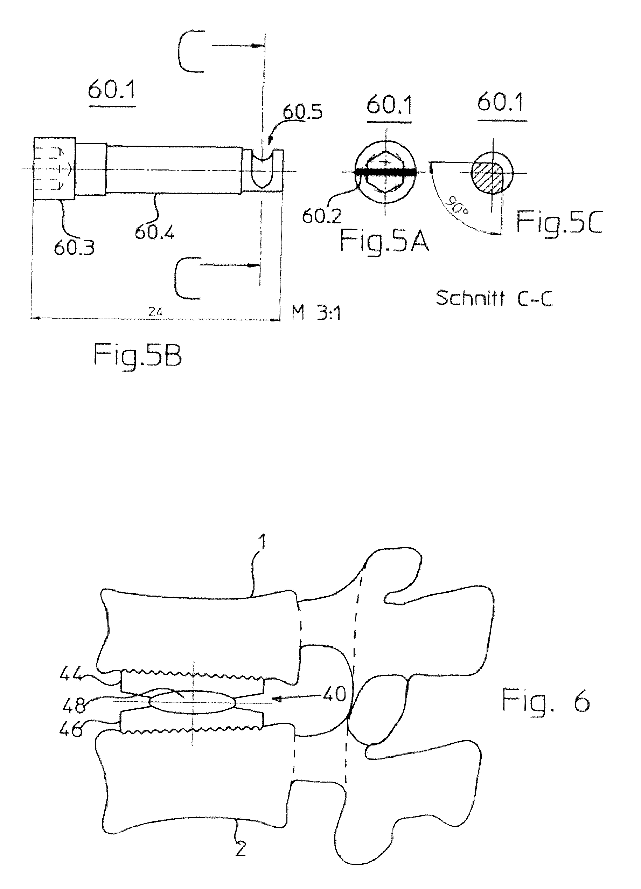

In Fig. 2 ist ein als Bandscheiben-Ersatz ausgebildetes Implantat 10 gezeigt. Ein solches Implantat 10 kann eine gesamte Bandscheibe oder einen Teil einer Bandscheibe ersetzen. Das Implantat 10 weist einen Implantatkörper 40 auf, der in den Fig. 3A und 3B gezeigt ist, ferner ein Fixierglied 50, das in den Fig. 4A bis 4D gezeigt ist, und im Weiteren eine Stellvorrichtung 60, die in den Fig. 5A bis 5C gezeigt ist.

Der eigentliche Implantat-Körper 40 ist blockähnlich. Er weist eine Vorderfläche 40.1, eine Hinterfläche 40.2 und zwei Seitenflächen 40.3 auf. Beim vorliegenden Ausführungsbeispiel ist die Vorderfläche 40.1 parallel zur Hinterfläche 40.2, und die beiden Seitenflächen 40.3 sind parallel zueinander, doch sind diese parallelen Anordnungen nicht zwingend. Im Weiteren weist der Implantat-Körper 10 eine Deckfläche 40.4 und eine Unterfläche 40.5 auf. Die Deckfläche 40.4 und die Unterfläche 40.5 sind nicht parallel sondern laufen von hinten nach vorn aufeinander zu, im wesentlichen konisch, obwohl sie auch in anderer Form aufeinander zu laufen oder parallel sein können. Je nach Höhenlage innerhalb der Wirbelsäule eines Patienten können die Vorderfläche 40.1 und die Hinterfläche 40.2 vertauscht sein, so dass die Deckfläche 40.4 und die Unterfläche 40.5 von vorn nach hinten voneinander weg streben.

Sowohl die Deckfläche 40.4 als auch die Unterfläche 40.5 sind nicht glatt, sondern weisen eine Struktur 40.6 auf, die beim vorliegenden Ausführungsbeispiel die Form von parallelen Rillen hat. Es sind auch anders geartete Strukturen möglich. Die Rillen der Struktur 40.6 sind, wenn das Implantat 10 einem Patienten eingesetzt ist, quer zur Längsmittelfläche des Patienten gerichtet.

Der Implantatkörper 40 weist mehrere Durchbrüche bzw. Ausnehmungen auf, nämlich zwei seitliche, vertikale Durchbrüche 40.7 auf, die im wesentlichen das Fixierglied 50 aufnehmen können, sowie einen mittigen, ebenfalls vertikalen Durchbruch 40.8. Im Weiteren weist der Implantatkörper 40 eine horizontale, sich zwischen den Seitenflächen 40.3 erstreckende Bohrung 40.9 zur Aufnahme eines Stellgliedes 60.1 der Stellvorrichtung 60 auf. Ferner weist der Implantat-Körper 40 eine vertikale Bohrung 40.10 zur Aufnahme eines Stiftes 42 auf, der dazu bestimmt ist, eine Sicherung für das sich in seiner finalen Aktivstellung befindliche Stellglied 60.1 zu bilden. Noch weitere Bohrungen, in Fig. 3A links der Bohrung 40.9, dienen als Angriff für ein Setzwerkzeug, dass heisst für ein während der Implantation zum Halten des Implantates 10 benötigten Werkzeuges. Diese Bohrungen sind nur für den eigentlichen Implantationsvorgang notwendig.

Der Implantatkörper 40 weist an seiner in Fig. 2 in der Zeichenebene oben liegenden Seitenfläche 40.3 einen Pfeil 40.11 mit einer Beschriftung ,LOCK' auf. Der Pfeil 40.11 und die Beschriftung ,LOCK' geben an, in welcher Richtung das Stellglied 60.1 zu drehen ist, um das Fixierglied 50 aus seiner Ruhelage weg auf seine Wirklage hin zu drehen. Der Implantatkörper 40 weist, ebenfalls an der in Fig. 2 in der Zeichenebene oben liegenden Seitenfläche 40.3, eine Körper-Markierung 40.12 auf. Diese Körper-Markierung 40.12 ist zur Zusammenwirkung mit einer Stellglied-Markierung 60.2 am Stellglied 60 bestimmt, und wenn die Stellglied-Markierung 60.2 auf die Körper-Markierung 40.12 ausgerichtet ist, nimmt das Fixierglied 50 seine extremale Wirklage ein. Es können auch mehrere Körper-Markierungen vorgesehen sein, die in Zusammenwirkung mit der Stellglied-Markierung 60.2 verschieden weit aus der Ruhelage ausgelenkte Lagen des Fixiergliedes 50 angeben. Dies ist deshalb interessant, weil das Fixierglied 50 nicht notwendigerweise in seine extremale Wirklage gebracht werden muss oder kann, um den Implantatkörper 40 zu fixieren. Die Markierungen können zum Beispiel durch Laserung aufgebracht werden.

Das Fixierglied 50 weist einen Mittelteil 50.1 mit einem hier quadratischen Schnitt auf, in dem eine zentrale Bohrung 50.2 angebracht ist, die zur Aufnahme des Stellgliedes 60 bestimmt ist, wie in den Fig. 4A - 4D gezeigt. Von zwei einander gegenüberliegenden Flächen des Mittelteiles 50.1 ragt je ein Ansatz in Form einer Lamelle 50.4 weg. Diese Lamellen 50.4 bilden den eigentlich wirksamen Bereich des Fixiergliedes 50. Jede der Lamellen 50.4 weist eine Schneidkante 50.5 auf. Die Lamellen 50.4 können gleich ausgebildet sein, so dass das Fixierelement 50 in der Ansicht gemäss Fig. 4B zentralsymmetrisch ist, doch ist eine gleiche Ausbildung nicht zwingend erforderlich. Anstelle zweier Lamellen kann das Fixierglied 40 auch nur eine Lamelle oder aber mehr als zwei Lamellen aufweisen.

Fig. 4B zeigt das Fixierglied 50 von der Seite in seiner Ruhelage. Die Ansätze bzw. Lamellen 50.5 würden sich hierbei innerhalb der seitlichen Durchbrüche 40.7 des Implantatkörpers 40 befinden. Bewegt man nun das Fixierglied 50 auf seine Wirklage zu, indem man es im Gegenuhrzeigersinn in Richtung des Pfeils A1 dreht, so werden dadurch die Lamellen 50.4 aus den Durchbrüchen 40.7 hinaus gedreht. Ihre Schneidkanten 50.5 laufen bei dieser Bewegung vor und schneiden sich in die angrenzenden Knochenteile 1, 2 bzw. Wirbelkörper ein bzw. durch die letzteren durch. Das Fixierglied 50 hat seine extremale Wirklage erreicht, wenn es um 90° gedreht worden ist. Bei anderen Ausführungsbeispielen können kleinere maximale Drehwinkel vorgesehen sein.

Jede der Lamellen 50.4 kann auch eine zweite Schneidkante 50.6 aufweisen. Diese Schneidkante ist beim Fixieren des Implantatkörpers 40, das heisst bei einer Drehung des Fixiergliedes 50 in Richtung des Pfeils A2 gemäss Fig. 4B, nachlaufend und hat hierbei keine Funktion. Soll aber das Implantat 10 aus dem Körper des Patienten entfernt werden, so muss zuvor das Fixierglied 50 wieder in seine Ruhelage gebracht werden. Weil sich der beim Einpflanzen des Implantats 10 durch das Fixierglied 50 verursachte Schneidspalt nach und nach mit neu gebildeter Knochensubstanz gefüllt hat, muss ein neuer Schneidspalt erzeugt werden. Dies geschieht durch die weiteren Schneidkanten 50.6, während das Fixierglied 50 im Uhrzeigersinn, das heisst gemäss Fig. 4B in Richtung des Pfeils A2, gedreht wird.

Das Stellglied 60.1, mit welchem das Fixierglied 50 verstellbar ist, ist in den Fig. 5A bis 5C dargestellt. Beim Stellglied 60.1 handelt es sich um eine Stellschraube oder -zapfen, mit einem die Stellglied-Markierung 60.2 aufweisenden Innensechskant-Schraubenkopf 60.3, einem Schraubenschaft 60.4, auf welchem das Fixierglied 50 bzw. mehrere Fixierglieder in gegenseitigem Abstand befestigt sind, und einem vorderen Endbereich 60.5, der zur Zusammenwirkung mit dem Stift 42 bestimmt ist.

Werden die oben beschriebenen Implantate 10 als Bandscheiben-Ersatz verwendet, so lassen sich damit Bandscheiben ganz oder teilweise ersetzen und dadurch Beschwerden der Patienten vermindern oder verhindern, allerdings durch einen teilweisen Verlust an Beweglichkeit. Dieser Verlust kann reduziert werden, wenn die Implantatkörper 40 geeignet ausgebildet werden, wie in Fig. 6 anhand eines Beispiels gezeigt. Zu diesem Zweck wird der Implantatkörper 40 in ein erstes Implantatkörper-Aussenteil 44 und ein zweites Implantatkörper-Aussenteil 46 aufgeteilt. Dazwischen wird ein Implantatkörper-Mittelteil 48 angeordnet ist, relativ zu welchem die Implantatkörper-Aussenteile 44, 46 im montierten Zustand begrenzt bewegbar sind. Hierzu kann von einander gegenüberstehenden Flächen eines Implantatkörper-Aussenteiles 44, 46 und des Implantatkörper-Mittelteiles 48 mindestens eine Fläche nach aussen konvex und vorzugsweise die andere Fläche nach aussen konkav ausgebildet sein. Das Implantatkörper-Mittelteil 48 kann auch elastisch sein.

Als Werkstoffe für Implantate gemäss der Erfindung eignen sich unter Anderem Titan, Chrom-Kobalt-Legierungen, Peek oder Keramiken wie Aluminiumoxyd oder Zirkonoxyd.

## Patentansprüche

1. Implantat (10) zum Ersetzen mindestens eines Teils eines Knochens oder Knorpels eines Patienten, umfassend
- einen Implantat-Körper (40) und
- eine Fixiereinrichtung mit mindestens einem Fixierglied (50), das aus einer Ruhelage in eine Wirklage bewegbar und dazu bestimmt ist, in der Wirklage in einen dem Implantat-Körper (40) benachbarten Knochen (1, 2) einzugreifen,
**dadurch gekennzeichnet,**
**dass** die Fixiereinrichtung eine Stellvorrichtung (60) aufweist, die
- bewegungsmässig mit dem Fixierglied (50) gekoppelt ist, und
- verstellbar ist zwischen
- einer Passivstellung, in der das Fixierglied (50) seine Ruhelage einnimmt, und
- einer Aktivstellung, in der das Fixierglied (50) eine Wirklage einnimmt.

2. Implantat (10) nach Anspruch **1,**
**dadurch gekennzeichnet,**
**dass** das Fixierglied (50) als Lamelle (50.4) mit mindestens einer Lamellenfläche ausgebildet ist, die eine Schneidkante (50.5) besitzt, welche dazu bestimmt ist, beim Bewegen des Fixiergliedes (50) vorzulaufen, um schneidend in den Knochen (1, 2) einzudringen.

3. Implantat (10) nach Anspruch **2,**
**dadurch gekennzeichnet,**
**dass** das die Lamelle (50.4) aufweisende Fixierglied (50) durch Drehung um eine, im Wesentlichen senkrecht zur Fläche der Lamelle (50.4) gerichtete, Achse drehbar ist.

4. Implantat (10) nach einem der Ansprüche **1** bis **3,**
**dadurch gekennzeichnet,**
**dass** die Fixiereinrichtung mindestens zwei, mit demselben Knochen (1, 2) in Eingriff bringbare Fixierglieder (50) umfasst.

5. Implantat (10) nach Anspruch **4,**
**dadurch gekennzeichnet,**
**dass** beide Fixierglieder (50) mittels derselben Stellvorrichtung (60) verstellbar sind.

6. Implantat (10) nach einem der Ansprüche **1** bis **5,**
**dadurch gekennzeichnet,**
**dass** die Stellvorrichtung (60) eine Stellschraube aufweist.

7. Implantat (10) nach einem der Ansprüche **1** bis **6,**
**dadurch gekennzeichnet,**
**dass** die Stellvorrichtung (60) eine Exzentneranordnung aufweist, über welche das Fixierglied (50) bewegbar ist.

8. Implantat (10) nach einem der Ansprüche **1** bis **7,**
**dadurch gekennzeichnet,**
**dass** der Implantat-Körper (40) und die Stellvorrichtung (60) Markierungen (40.11, 60.2) aufweisen, deren relative Stellung die Lage des Fixiergliedes (50) angeben.

9. Implantat (10) nach einem der Ansprüche **1** bis **8,**
**dadurch gekennzeichnet,**
**dass** sie mindestens ein Sicherungselement (42) aufweist, um das mindestens eine Fixierglied (50) mittelbar oder unmittelbar in seiner Wirklage zu sichern.

10. Implantat (10) nach einem der Ansprüche **1** bis **9,**
**dadurch gekennzeichnet,**
**dass** es eine weitere Fixiereinrichtung aufweist, deren mindestens eines Fixierglied dazu bestimmt ist, in einen weiteren angrenzenden Knochen einzugreifen.

11. Implantat (10) nach einem der Ansprüche **1** bis **10,**
**dadurch gekennzeichnet,**
**dass** der Implantat-Körper (40) und ggfs. das mindestens eine Fixierglied (50) eine äussere Fläche mit einer Struktur (40.6) aufweisen.

12. Implantat (10) nach einem der Ansprüche **1** bis **11,**
**dadurch gekennzeichnet,**
**dass** der Implantat-Körper (40) an der dem angrenzenden Knochen (1, 2) zugewandten Deckfläche (40.4) und/oder Unterfläche (40.5) eine Struktur (40.6) aufweist, beispielsweise mit Rillen, die im montierten Zustand vorzugsweise quer zur Richtung der Lamellen (50.4) verlaufen.

13. Implantat (10) nach einem der Ansprüche **1** bis **12,**
**dadurch gekennzeichnet,**
**dass** die Höhen des Implantat-Körpers (40) entsprechend dem örtlichen Abstand der im montierten Zustand des Implantates (10) benachbarten Knochen (1, 2) gewählt wird.

14. Implantat (10) nach einem der Ansprüche **1** bis **13,**
**dadurch gekennzeichnet,**
**dass** der Implantat-Körper (40) Ausbrüche oder Durchbrüche (40.8) aufweist.

15. Implantat (10) nach Anspruch **1,**
**dadurch gekennzeichnet,**
**dass** der Implantat-Körper (40) in ein erstes Implantatkörper-Aussenteil (44) und ein zweites Implantatkörper-Aussenteil (46) aufgeteilt ist, zwischen denen ein Implantatkörper-Mittelteil (48) angeordnet ist, relativ zu welchem die Implantatkörper-Aussenteile (44, 46) im montierten Zustand begrenzt bewegbar sind.

16. Implantat (10) nach Anspruch **15,**
**dadurch gekennzeichnet,**
**dass** von einander gegenüberstehenden Flächen eines Implantatkörper-Aussenteiles (44, 46) und des Implantatkörper-Mittelteiles (48) mindestens eine Fläche nach aussen konvex und vorzugsweise die andere Fläche nach aussen konkav ausgebildet ist.

17. Implantat (10) nach einem der Ansprüche **15** bis **16,**
**dadurch gekennzeichnet,**
**dass** das Implantatkörper-Mittelteil (48) elastisch ist.

18. Implantat (10) nach einem der Ansprüche **1** bis **17,**
**dadurch gekennzeichnet,**
**dass** es ein Bandscheiben-Implantat zum Ersatz mindestens eines Teils einer Bandscheibe ist, und
- **dass** die angrenzenden Knochen aufeinanderfolgende Wirbelkörper sind.
